# EUROPEAN PATENT APPLICATION

(11) **EP 2 904 996 A1**
(43) Date of publication of application: **12.08.2015**
(21) Application number: 15154641.3
(22) Date of filing: 11.02.2015
(51) Int. Cl.: A61F 5/443

(54) **Seal for use with a stoma**

(30) Priority: 11.02.2014 GB 201402289
(71) Applicant: T.G. Eakin Limited, Comber, County Down BT23 5QY (GB)
(72) Inventor: Mahood, Benjamin, Comber, Down BT23 5QY (GB)
(74) Representative: Armstrong, Rosemary

(57) **Abstract**

The invention provides a seal (1) for fitting around a stoma, comprising a single, flexible, adhesive material having a body (3) in the shape of a split ring, having a first face (5) for attaching to a peristomal surface, a second face (7) for attaching to an ostomy appliance, a wall (9) extending from the first face to the second face and providing an outer wall surface (11) and an inner wall surface (13) which defines an aperture for receiving the stoma, and a first end (15) and a second end (17) spaced apart to define an opening which extends from the outer wall surface to the inner wall surface, wherein the single material and oval, split ring shape of the seal causes the seal to be manipulable to fit around the stoma.

## Description

The invention relates to seals for use with stomas, for example stomas of the colon, to provide a barrier between skin surrounding a stoma and output from the stoma.

For a number of medical reasons, it may be necessary to provide a person with an artificial stoma, i.e. an artificial opening in the body. A well-known form of an artificial stoma is a colostomy, where an opening is surgically created in the large intestine. This is formed by bisecting the colon and drawing an end of the colon through an incision in the abdominal wall. The abdominal wall is stitched around the protruding end of the colon, forming an artificial stoma of the colon through the abdominal wall. An ostomy appliance, such as a pouch, is generally attached to the body sealing around the stoma to collect output from the colon via the stoma.

A common problem with stomas is irritation of the skin around the stoma, due to, for example, output from the stoma coming into contact with the skin. This can be reduced by providing a seal around the stoma. The seal is placed around the stoma to cover the skin and provides a barrier between the skin and any output from the stoma. A pouch may then be adhered to the seal. Known seals comprise an annular shape or comprise a strip of material that can be placed around the stoma.

The shape and size of a stoma and the location of a stoma with respect to the body surface varies from person to person. The surface of the skin surrounding a stoma will also vary, for example the skin surface can be rough or smooth and may or may not comprise folds or wrinkles. Such variation can lead to an inadequate fit of known seals around a stoma, providing an inadequate barrier between output from the stoma and the skin around the stoma.

The present invention seeks to provide a seal which achieves an improved fit around a stoma and an improved barrier between stoma output and skin around the stoma. Due to the significant personal and medical concerns caused by stoma leakage, any appreciable improvement in this area is of great importance to the quality of life of stoma patients.

According to the invention there is provided a seal for fitting around a stoma, comprising a single flexible, adhesive, material, having a body formed in the shape of a split ring, comprising
a first face for attaching to a peristomal surface,
a second face for attaching to an ostomy appliance,
a wall extending from the first face to the second face and providing an outer wall surface and an inner wall surface which defines an aperture for receiving the stoma, and
a first end and a second end spaced apart to define an opening which extends from the outer wall surface to the inner wall surface,
wherein the single material and oval, split ring shape of the seal causes the seal to be manipulable to fit around the stoma.

It has been found that providing a seal of a single, flexible material facilitates improved fitting of the seal around stomas of various sizes and shapes. An improved barrier between skin around the stoma and output from the stoma has been achieved, providing medical and personal benefits to ostomy patients.

The outer wall surface may form a substantially oval shape. The outer wall surface may form a substantially circular shape.

The inner wall surface may form a substantially oval aperture. The inner wall surface may form a substantially circular aperture.

The seal may be stretchable to fit around the stoma. The seal may be mouldable to substantially conform with the stoma. The body of the seal may be mouldable to substantially conform with a peristomal surface.

The seal may comprise any suitable flexible, adhesive skin barrier material. The seal may comprise a flexible, adhesive hydrocolloid material.

The first and second faces of the seal may each be provided with a protective covering such as a release liner.

The first and second ends may comprise flat end walls having rounded corners. The first and second ends may comprise substantially rounded end walls. The first and second ends may comprise substantially flat end walls. The end walls may be substantially parallel. The first and second ends may, in use, overlap or abut to form a seal around the stoma. The first and second ends may, in use, overlap at a position around the stoma where output from the stoma is most likely to be present. The first end and/or the second end of the seal may be tearable to remove excess seal.

The first and second faces may be generally flat. The first and second faces may be shaped to fit to a peristomal surface. The wall may be generally perpendicular to the first and second faces. The wall may be sloped with respect to the first and second faces.

The seal may made in various sizes, for example to fit around stomas of different types.

An embodiment of the invention will now be described by way of example only, with reference to the accompanying drawings, in which:

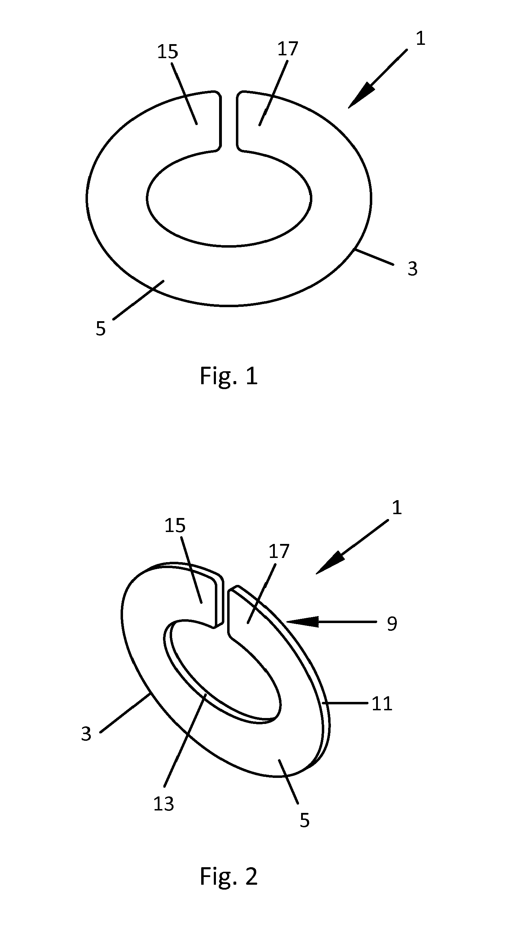

Figure 1 is a plan view of a seal according to the invention;
Figure 2 is a perspective view of the seal of Figure 1;
Figure 3 is a perspective view of the seal of Figure 1 and Figure 2 shown in conjunction with a stoma and a peristomal surface, and
Figure 4 is a side view of the seal of Figures 1 to 3, shown in conjunction with the stoma, the peristomal surface and an ostomy appliance.

Referring to the Figures, the seal 1 comprises a body 3 formed in the shape of an oval, split ring. It will be appreciated that, in other embodiments of the invention, the seal may circular or may have an outer circular shape and an inner oval shape or may have an outer oval shape and an inner circular shape.

The body 3 comprises a first face 5 for attaching to a peristomal surface, a second face 7 for attaching to an ostomy appliance and a wall 9 extending from the first face 5 to the second face 7. The wall 9 provides a substantially oval outer wall surface 11 and an inner wall surface 13 which defines a substantially oval aperture for receiving a stoma. The body 3 further comprises a first end 15 and a second end 17 which are spaced apart to define an opening which extends from the outer wall surface 11 to the inner wall surface 13. The first and second ends, 15, 17 each comprise a flat end wall having rounded corners where the end wall attaches to the outer and inner wall surfaces 11, 13. It will be appreciated that, in other embodiments of the invention, the end walls may be differently shaped, e.g. may be substantially rounded end walls or substantially flat end walls.

The seal 1 comprises a single, flexible, adhesive material which may be any suitable flexible, adhesive skin-barrier material, such as a flexible, adhesive hydrocolloid material. The first and second faces 5, 7 can be provided with a protective covering such as a release liner. The seal 1 may made in various sizes, to fit around stomas of different types. In this embodiment, the seal 1 has a thickness of approximately 2.8mm, a major axis length of approximately 85mm and a minor axis length of approximately 65mm, the aperture has a major axis length of approximately 49mm and a minor axis length of approximately 30mm, and the first and second ends are spaced apart by approximately 5mm.

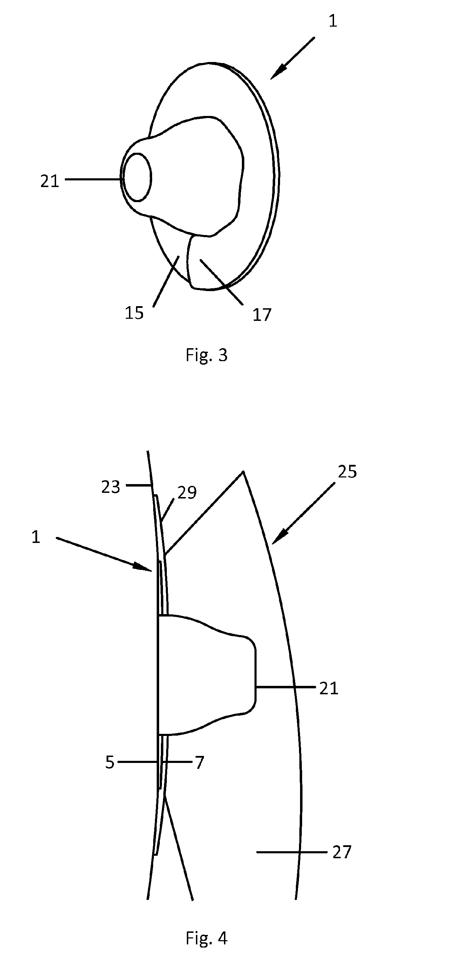

To use the seal 1, it is first removed from any release liners and may be warmed slightly in the hands to enhance its stretchability and mouldability. Referring to Figure 3, the seal 1 is placed around a stoma 21, such that the stoma 21 is received in the aperture of the seal 1 and the first face 5 lies on a peristomal skin surface 23. The seal 1 is manipulable to fit closely around the stoma, in particular the seal is mouldable around the stoma 21 and onto the peristomal surface 23, to substantially conform with the stoma 21 and the peristomal surface 23, as required. The substantially oval shape of the seal 1 allows it to be more easily manipulable to fit better the shape of the stoma 21. The opening provided in the seal 1 allows user expansion and contraction of the circumference of the body of the seal 1 to fit better around the stoma. The first and second ends 15, 17 of the seal 1 are overlapped, preferably at a position around the stoma 21 where output is most likely to be present, e.g. where skin folds or crevices are present. The seal 1 is then pressed against the peristomal surface 23 to enhance adherence of the seal 1 to the surface 23. Should excess seal be present at the overlap, a portion of one end or of both ends 15, 17 is removed by tearing.

Referring to Figure 4, an ostomy appliance such as a pouch 25 is then fitted to the seal 1. The pouch 25 comprises a bag 27 and a ring flange 29 attached to the bag. The ring flange 29 is placed over the stoma 21 and is adhered to the second face 7 of the seal 1 and also to the peristomal surface 23. Output from the stoma 21 is collected in the bag 27. Any output which leaks between the ring flange 29 and the stoma 21 will encounter the seal 1. The seal 1 acts as a barrier between the skin of the peristomal surface 23 and the output from the stoma 21. The hydrocolloid material of the seal 1 will absorb moisture from the output further protecting the peristomal skin surface.

It can be seen that the seal of the invention is used, on its own, with an ostomy appliance. No complicated series of skin barrier flanges, seals and ostomy product flanges is required to provide a barrier between the skin around the stoma and output from the stoma. It has been found that providing a seal comprising a single, flexible, adhesive material, with no separate layers, allows a user to much more easily manipulate the seal to fit closely around the stoma 21. It has also been found that providing a seal having an outer and inner oval, split ring shape makes it easier to fit around stomas, particularly stomas of various shapes and sizes.

## Claims

1. A seal (1) for fitting around a stoma, comprising a single, flexible, adhesive material having a body formed in the shape of a split ring, comprising
a first face (5) for attaching to a peristomal surface,
a second face (7) for attaching to an ostomy appliance,
a wall extending (9) from the first face to the second face and providing an outer wall surface (11) and an inner wall surface (13) which defines an aperture for receiving the stoma, and
a first end (15) and a second end (17) spaced apart to define an opening which extends from the outer wall surface to the inner wall surface,
wherein the single material and oval, split ring shape of the seal causes the seal to be manipulable to fit around the stoma.

2. A seal according to claim 1 in which the outer wall surface (11) forms a substantially oval shape.

3. A seal according to claim 1 in which the outer wall surface (11) forms a substantially circular shape.

4. A seal according to any preceding claim in which the inner wall surface (13) forms a substantially oval aperture.

5. A seal according to any of claims 1 to 3 in which the inner wall surface (13) forms a substantially circular aperture.

6. A seal according to any preceding claim in which the seal is stretchable to fit around the stoma.

7. A seal according to any preceding claim in which the seal is mouldable to substantially conform with the stoma and is mouldable to substantially conform with a peristomal surface.

8. A seal according to any preceding claim in which the seal comprises a flexible, adhesive hydrocolloid material.

9. A seal according to any preceding claim in which the first and second ends (15, 17) comprise flat end walls having rounded corners.

10. A seal according to any of claims 1 to 8 in which the first and second ends (15, 17) comprise substantially rounded end walls.

11. A seal according to any of claims 1 to 8 in which the first and second ends (15, 17) comprise substantially flat end walls.

12. A seal according to any preceding claim in which the first and second ends (15, 17), in use, overlap or abut to form a seal around the stoma.

13. A seal according to any preceding claim in which the first and second faces (5, 7) are generally flat.

14. A seal according to any of claims 1 to 12 in which the first and second faces (5, 7) are shaped to fit to a peristomal surface.

15. A seal according to any preceding claim in which the wall (9) is sloped with respect to the first and second faces (5, 7).
